Europäisches Patentamt

European Patent Office

Office européen des brevets

(11) Publication number: **0 307 026 B1**

(12) **EUROPEAN PATENT SPECIFICATION**

(45) Date of publication of patent specification: **18.03.92**   (51) Int. Cl.⁵: **C07C 11/08**, C07C 2/36

(21) Application number: **88201804.7**

(22) Date of filing: **24.08.88**

(54) **Process for the preparation of a butene.**

(30) Priority: **28.08.87 GB 8720379**

(43) Date of publication of application:
**15.03.89 Bulletin 89/11**

(45) Publication of the grant of the patent:
**18.03.92 Bulletin 92/12**

(84) Designated Contracting States:
**BE DE FR GB IT NL SE**

(56) References cited:
**EP-A- 0 170 311**
**EP-A- 0 188 830**

(73) Proprietor: **SHELL INTERNATIONALE RE-
SEARCH MAATSCHAPPIJ B.V.
Carel van Bylandtlaan 30
NL-2596 HR Den Haag(NL)**

(72) Inventor: **Drent, Eit
Badhuisweg 3
NL-1031 CM Amsterdam(NL)**

# Description

The invention relates to a process for the preparation of a butene by dimerization of ethylene.

According to EP-188830 butenes can be prepared by dimerization of ethylene, ethylene being contacted in an aprotic organic solvent with a catalytic system formed by combining a palladium compound, an acid with a pKa of less than 2, except hydrohalogenic acids, and a ligand containing at least two atoms of Group 5a of the Periodic Table of the Elements, which are connected through a chain comprising 2-6 carbon atoms. The experiments so far carried out with this known process yielded mixtures of 1-butene and 2-butene which usually predominantly contained 2-butene.

1-Butene is a monomer for the preparation of a large variety of polymers and copolymers and a process for the preparation of butenes which predominantly consist of 1-butene is therefore of great importance.

It is an object of the present invention to prepare 1-butene with a very high selectivity and a high rate of dimerization.

Accordingly, the invention provides a process for the preparation of a butene by dimerization of ethylene in which process ethylene is contacted in an aprotic solvent with a catalytic system, characterized in that 1-butene is prepared by using a catalytic system which is obtainable by combining the following components:-

component (a) -

a palladium compound,

component (b) -

an anion of an acid having a pKa of less than 2, measured at 18 °C in aqueous solution, with the proviso that said acid is not a hydrohalogenic acid, and

component (c) -

a compound containing one phosphorus and one or more nitrogen atoms which atoms bear no hydrogen atoms, and in which compound each nitrogen atom is connected to the phosphorus atom by an organic bridging group containing at least one carbon atom in the bridge.

It has, surprisingly, been found that the process according to the present invention allows a very high selectivity to 1-butene, which may be higher than 90%, with formation of only traces of alkenes having more than four carbon atoms per molecule or none of it at all.

The "selectivity to 1-butene", expressed in a percentage, is defined herein as 100 x a:b, in which "a" is the amount of 1-butene and "b" the sum of the amounts of 1-butene and 2-butene that has been formed by the dimerization.

Both homogeneous and heterogeneous palladium compounds may be used in the process according to the present invention. Homogeneous compounds are preferred. Suitable homogeneous compounds are palladium salts of nitric acid and sulphuric acid. Palladium salts of alkanoic acids having in the range of from 1 to 12 carbon atoms per molecule are preferred.

Salts of hydrohalogenic acids may, in principle, be used as well, but they have the drawback that the halogen ion may have a corrosive effect.

A palladium compound used by preference is palladium acetate. Moreover, palladium complexes may be used, for instance palladium acetylacetonate, tetrakistriphenylphosphinepalladium, bis(tri-o-tolylphosphine)palladium acetate and bis-(triphenylphosphine)palladium sulphate. Palladium on charcoal and palladium bound to an ion exchanger - for instance an ion exchanger comprising sulphonic acid groups - are examples of suitable heterogeneous catalysts. Component (a) may also be a mixture of palladium compounds.

The acid with a pKa of less than 2 used in the process of the invention may comprise organic acids such as carboxylic acids and organic sulphonic acid as well as inorganic acids, which preferably have a non-coordinating anion by which is meant that little or no interaction takes place between the palladium and the anion. Typical examples of such anions are $PF_6^-$, $SbF_6^-$, $BF_4^-$ and $ClO_4^-$.

Acids preferably used are, for instance, perchloric acid, sulphuric acid, sulphonic acids and those acids that can be formed possibly in situ, by interaction of a Lewis acid such as, for example, $BF_3$, $AsF_5$, $SbF_5$, $PF_5$, $TaF_5$ or $NbF_5$ with a Broensted acid such as, for example, a hydrohalogenic acid, in particular HF, fluorosulphonic acid, phosphoric acid or sulphuric acid. Specific examples of the last-named type of acids are fluorosilicic acid, $HPF_4$, $HSbF_4$ and $HBF_4$, which is preferred. Typical sulphonic acids that can be used are fluorosulphonic acid, chlorosulphonic acid and the organic sulphonic acids such as, for example, methanesulphonic acid and 2-hydroxypropanesulphonic acid. Sulphonic acids are preferred, in particular p-toluenesulphonic acid and trifluoromethanesulphonic acid.

Carboxylic acids which may be used are for example trifluoroacetic acid, trichloroacetic acid and dichloroacetic acid.

Component (b) may also be a mixture of different anions of acids having a pKa of less than 2.

Component (b) is preferably incorporated in the catalytic system in the form of an acid and/or as a non-noble transition metal salt. The use of such salts is preferred because they are considerably less corrosive than the acids. When component (b) is used in the form of a non-noble transition metal salt, preference is given to a copper salt, in particular a cupric salt.

If desired, components a) and b) may be combined in a single compound. An example of such a compound is the complex $Pd(CH_3CN)_2(O_3S-C_6H_4-CH_3)_2$, which can be prepared by reaction in acetonitrile of either palladium chloride with silver paratosylate, or palladium acetate with para-toluenesulphonic acid.

The component c) preferably used in the catalyst compositions is a phosphorus/nitrogen compound which may be regarded as being derived from triphenylphosphine by replacing at least one of the phenyl groups therein by a group containing one or more nitrogen atoms. The organic bridging group through which the phosphorus atom is connected to a nitrogen atom preferably contains not more than three carbon atoms in the bridge. Special preference is given to phosphorus/nitrogen compounds which may be regarded as being derived from triphenylphosphine by replacing no more than one of the phenyl groups therein by a group containing only one nitrogen atom.

Examples that may be mentioned of phosphorus/nitrogen compounds which may be regarded as being derived from triphenylphosphine by replacing one of the phenyl groups therein by a group containing a single nitrogen atom and in which compounds the bridging group which connects the phosphorus to the nitrogen atom contains a single carbon atom, are:-
2-pyridyl-diphenylphosphine,
2-(diphenylphosphino)-1-methyl-1-pyrrole,
1-(diphenylphosphino)-N,N-diphenylformamide, and
1-[(diphenylphosphino)methyl]-2,5-pyrrolidinedione.

Examples that may be mentioned of phosphorus/nitrogen compounds which can be regarded as being derived from triphenylphosphine by replacing one of the phenyl groups therein by a group containing one nitrogen atom, and in which compounds the bridging group which connects the phosphorus atom with the nitrogen atom contains two carbon atoms, are:-
1-[(2-diphenylphosphino)ethyl]-2-pyrrolidinone,
2-(diphenylphosphino)-N,N-diphenylacetamide,
(2-isocyanoethyl)diphenylphosphine,
8-(diphenylphosphino)quinoline,
ortho-(diphenylphosphino)-N,N-dimethylaniline,
2-(diphenylphosphino)-triethylamine,
2-[(diphenylphosphino)methyl]pyridine, and
N-[(diphenylphosphino)methyl]diethylamine.

Examples that may be mentioned of phosphorus/nitrogen compounds which can be regarded as being derived from triphenylphosphine by replacing one of the phenyl groups therein by a group containing one nitrogen atom, and in which compounds the bridging group which connects the phosphorus atom to the nitrogen atom contains three carbon atoms are:-
2-cyanoethyl-diphenylphosphine,
2-[2-(diphenylphosphino)ethyl]-pyridine,
2-[(diphenylphosphino)methyl]-N,N-dimethylbenzamine,
2-(diphenylphosphino)-N,N-dimethylbenzenemethanamine,
2-(diphenylphosphino)-N,N-diethylbenzamine, and
3-(diphenylphosphino)-N,N-dimethylpropionamide.

Examples that may be mentioned of phosphorus/nitrogen compounds which can be regarded as being derived from triphenylphosphine by replacing two or three phenyl groups therein by a group containing one nitrogen atom, are bis(2-pyridyl)phenylphosphine, and tris(2-cyanoethyl)phosphine.

Component (c) may be a mixture of two different components (c).

Other examples of suitable components (c) are:-
di(p-methoxyphenyl)-2-pyridylphosphine,
di(p-tolyl)-2-pyridylphosphine,
di(o-methoxyphenyl)-2-pyridylphosphine,
di(o-chlorophenyl)-2-pyridylphosphine,
di(methoxyphenyl)-2-pyridylphosphine,
di(m-chlorophenyl)-2-pyridylphosphine,
di(p-methoxyphenyl)-3-pyridylphosphine,
di(p-tolyl)-3-pyridylphosphine,
di(o-methoxyphenyl)-3-pyridylphophine,
di(o-chlorophenyl)-3-pyridylphosphine,
di(m-methoxyphenyl)-3-pyridylphosphine,
di(m-chlorophenyl)-3-pyridylphosphine,
di(p-methoxyphenyl)-4-pyridylphosphine,
di(p-tolyl)-4-pyridylphophine,
di(o-methoxyphenyl)-4-pyridylphosphine,
di(o-chlorophenyl)-4-pyridylphosphine,
di(m-methoxyphenyl)-4-pyridylphosphine,
di(m-chlorophenyl)-4-pyridylphosphine,
diphenyl(3-methoxy-2-pyridyl)phosphine,
diphenyl(4-methoxy-2-pyridyl)phosphine,
diphenyl(4-chloro-2-pyridyl)phosphine,
diphenyl(2-methoxy-3-pyridyl)phosphine,
diphenyl(4-methoxy-3-pyridyl)phosphine,
diphenyl(4-chloro-3-pyridyl)phosphine,
diphenyl(3-methoxy-4-pyridyl)phosphine,
diphenyl(3-chloro-4-pyridyl)phosphine,
diphenyl(5-chloro-4-pyridyl)phosphine,
diphenyl(5-methoxy-4-pyridyl)phosphine,
di(p-tolyl)(3-methoxy-4-pyridyl)phosphine,
di(p-tolyl)(3-chloro-4-pyridyl)phosphine,
di(m-methoxyphenyl)(3-chloro-4-pyridyl)phosphine,
di(m-methoxyphenyl)(3-methoxy-4-pyridyl)-phosphine,
di(m-chlorophenyl)(3-methoxy-4-pyridyl)phosphine,
di(p-tolyl)(3-methoxy-2-pyridyl)phosphine,
di(p-tolyl)(3-chloro-2-pyridyl)phosphine,
di(m-methoxyphenyl)(3-chloro-2-pyridyl)phosphine,
di(m-methoxyphenyl)(3-methoxy-2-pyridyl)-phosphine,

di(m-tert.-butoxyphenyl)(3-chloro-2-pyridyl)-phosphine,
di(m-tert.-butoxyphenyl)(3-methoxy-2-pyridyl)-phosphine,
di(m-tert.-butoxyphenyl)(3-chloro-4-pyridyl)-phosphine,
di(m-tert.-butoxyphenyl)(3-methoxy-4-pyridyl)-phosphine,
di(m-tert.-butoxyphenyl)(2-methoxy-3-pyridyl)-phsophine,
di(m-tert.-butoxyphenyl)(2-chloro-3-pyridyl)-phosphine,
di(m-chlorophenyl)(2-methoxy-3-pyridyl)phosphine,
di(m-chlorophenyl)(2-chloro-3-pyridyl)phosphine,
di(o-chlorophenyl)(2-methoxy-3-pyridyl)phosphine,
di(p-methoxyphenyl)-2-pyrimidinylphosphine,
di(p-tolyl)-2-pyrimidinylphosphine,
di(o-methoxyphenyl)-2-pyrimidinylphosphine,
di(o-chlorophenyl)-2-pyrimidinylphosphine,
di(m-methoxyphenyl)-2-pyrimidinylphosphine,
di(p-methoxyphenyl)-2-pyridazinylphosphine,
di(p-tolyl)-2-pyridazinylphosphine,
di(o-methoxyphenyl)-2-pyridazinylphosphine,
di(o-chlorophenyl)-2-pyridazinylphosphine,
di(m-methoxyphenyl)-2-pyridazinylphosphine,
di(p-methoxyphenyl)(3-methoxy-2-pyrimidinyl)-phosphine,
di(p-tolyl)(3-methoxy-2-pyridinyl)phosphine,
di(o-chlorophenyl)(3-chloro-2-pyrimidinyl)-phosphine,
di(m-methoxyphenyl)(3-chloro-2-pyrimidinyl)-phosphine,
di(p-tolyl)(4-methoxy-3-pyridazinyl)phosphine,
di(p-methoxyphenyl)(4-methoxy-3-pyridazinyl)-phosphine,
di(o-chlorophenyl)(4-methoxy-3-pyridazinyl)-phosphine,
phenyl-di(3-methoxy-2-pyridyl)phosphine,
phenyl-di(4-methoxy-2-pyridyl)phosphine,
phenyl-di(4-chloro-2-pyridyl)phosphine,
phenyl-di(2-methoxy-3-pyridyl)phosphine,
phenyl-di(4-methoxy-3-pyridyl)phosphine,
phenyl-di(4-chloro-3-pyridyl)phosphine,
phenyl-di(3-methoxy-4-pyridyl)phosphine,
phenyl-di(3-chloro-4-pyridyl)phosphine,
phenyl-di(5-chloro-4-pyridyl)phosphine,
phenyl-di(5-methoxy-4-pyridyl)phosphine,
phenyl-di(3-methoxy-2-pyrimidinyl)phosphine,
phenyl-di(3-chloro-2-pyrimidinyl)phosphine,
phenyl-di(4-methoxy-2-pyrimidinyl)phosphine,
phenyl-di(4-methoxy-3-pyridazinyl)phosphine and
phenyl-di(4-chloro-3-pyridazinyl)phosphine.

An example of a phosphorus/nitrogen compound which can be regarded as being derived from triphenylphosphine by replacing one of the phenyl groups therein by a group containing more than one nitrogen atom that may be mentioned is 2-(diphenylphosphino)-1,3,5-triazine.

Attractive catalyst compositions according to the invention can be composed by using any one of the following five phosphorus/ nitrogen compounds as component c):-

2-cyanoethyl-diphenylphosphine,
tris(2-cyanoethyl)phosphine,
2-pyridyl-diphenylphosphine,
bis(2-pyridyl)phenylphosphine, and
3-(diphenylphosphino)-N,N-dimethylpropionamide.

If desired, components a) and c) may be combined in a single compound. Examples of such compounds are bis(diphenyl-2-pyridylphosphine)-palladium acetate, tetrakisdiphenyl-2-pyridylphosphine palladium, bis-(di-o-tolylpyridyl)-phosphinepalladium acetate and bis-(diphenylpyridyl)phosphinepalladium sulphate.

Preferred solvents for use in the process according to the present invention are ethers and esters. Examples of ethers are 2,5,8-trioxanonane (also referred to as "diglyme"), anisole, diphenyl ether, diisopropyl ether, methyl butyl ether, 1,4-dioxane and tetrahydrofuran. Examples of esters are methyl benzoate, methyl acetate and gamma-butyrolactone. Further examples of suitable solvents are sulphoxides, such as dimethyl sulphoxide and diethyl sulphoxide; sulphones, such as diisopropyl sulphone, tetrahydrothiophene 1,1-dioxide (also referred to as "sulfolane"), 2-methylsulfolane, 3-methylsulfolane and 2-methyl-4-butylsulfolane; aromatic hydrocarbons, such as benzene, toluene and the three xylenes; halogenated hydrocarbons, such as chloroform, 1,2-dichloroethane, perfluoroalkanes, chlorobenzene and the three dichlorobenzenes; nitro compounds such as nitromethane and nitrobenzene.

The amount of palladium used per litre of solvent, the molar ratio of component (b) to component (a) and the molar ratio of component (c) to component (a) that may be used in the process according to the present invention are not critical and may vary within wide ranges, also below and above those specified hereinafter. Preferably, use is made of an amount of palladium in the range of from 0.1 to 100 milligram -atom per 1 solvent, a molar ratio of component (b) to component (a) in the range of from 1 to 50 and a molar ratio of component (c) to component (a) in the range of from 0.1 to 10.

The process according to the present invention may be carried out at a temperature and a pressure which are not critical and may vary within wide ranges. Preferably, the process is carried out at a temperature in the range of from 20 °C to 150 °C and a pressure in the range of from 5 to 200 bar.

During the process according to the present invention one or more of the three components of the catalyst composition may be supplied, continuously or intermittently, to compensate for possible losses thereof, if any such losses might occur.

1-Butene may be isolated from the reaction mixture obtained by the process according to the present invention in any desired manner, for example by means of distillation, leaving the catalytic system in the residue thus formed. The catalytic system may be reused, if desired.

The following examples further illustrate the invention.

## Example 1

A 250 ml magnetically stirred Hastelloy C autoclave ("Hastelloy" is a trade mark) was charged with diglyme (50 ml), palladium acetate (0.5 mmol), 2-pyridyldiphenylphosphine (0.6 mmol) and cupric trifluoromethylsulphonate (1.5 mmol), flushed with ethylene and pressurized with ethylene until a pressure of 40 bar was attained. Then, the autoclave was heated to a temperature of 60 °C and kept at this temperature for 5 h. At the end of this period the pressure in the autoclave was 16 bar; the autoclave was allowed to adopt ambient temperature and the contents were analyzed by gas/liquid chromatography.

The rate of dimerization of ethylene was 100 mol per gram-atom palladium per h, with selectivity to 1-butene of 90%.

## Comparative Experiment A

An experiment was carried out as described in Example 1 with the difference that 2-pyridyl-diphenylphosphine (0.6 mmol) was replaced with triphenylphosphine (1.0 mmol). The autoclave was heated to a temperature of 60 °C and kept at this temperature for 1.5 h. The rate of dimerization of ethylene was 450 mol per gram-atom palladium per h, with a selectivity to 1-butene of 14%. This selectivity is considerably lower than that observed in Example 1.

## Comparative Experiment B

An experiment was carried out as described in Example 1 with the difference that 2-pyridyl-diphenylphosphine (0.6 mmol) was replaced with 2,2'-bipyridyl (0.6 mmol).

The rate of dimerization of ethylene was 40 mol per gram-atom palladium per h, with a selectivity to 1-butene of 86%. Comparison with Example 1, where 2-pyridyldiphenylphosphine was used according to the invention, shows that in this Example a reaction rate was observed which is 2.5 times higher than that observed in this Comparative Experiment B where 2,2'-bipyridyl was used not according to the invention.

## Comparative Experiment C

An experiment was carried out as described in Example 1 with the difference that palladium acetate (0.5 mmol) was replaced with ruthenium triacetyl-acetonate (0.5 mmol), and that the autoclave was heated to a temperature of 120 °C instead of 60 °C.

The rate of dimerization of ethylene was only 50 mol per gram-atom ruthenium per h, with a selectivity to 1-butene of only 22%.

## Example 2

An experiment was carried out as described in Example 1 with the difference that 2-pyridyl-diphenylphosphine (0.6 mmol) was replaced with 3-(diphenylphosphino)-N,N-dimethylpropionamide. The autoclave was heated to a temperature of 60 °C and kept at this temperature for 3 h.

The rate of dimerization of ethylene was 165 mol per gram-atom palladium per h, with a selectivity to 1-butene of 84%.

## Example 3

An experiment was carried out as described in Example 1 with the difference that 2-pyridyl-diphenylphosphine (0.6 mmol) was replaced with 2-cyanoethyldiphenylphosphine (0.6 mmol). The autoclave was heated to a temperature of 60 °C and kept at this temperature for 1 h.

The rate of dimerization of ethylene was 450 mol per gram-atom palladium per h, with a selectivity to 1-butene of 68%.

## Example 4

An experiment was carried out as described in Example 3 with the difference that cupric trifluoromethylsulphonate (1.5 mmol) was replaced with trifluoromethanesulphonic acid (3 mmol).

The rate of dimerization of ethylene was 510 mol per gram-atom palladium per h, with a selectivity to 1-butene of 72%.

## Comparative Experiment D

An experiment was carried out as described in Example 4 with the difference that 2-cyanoethyl-diphenylphosphine (0.6 mmol) was replaced with triphenylphosphine (1.2 mmol).

The rate of dimerization of ethylene was 800 mol per gram-atom palladium per h, with a selectivity to 1-butene of 14%. This selectivity is considerably lower than that observed in Example 4.

## Example 5

An experiment was carried out as described in Example 1 using the following materials: methyl benzoate (50 ml), palladium acetate (0.1 mmol), 2-cyanoethyldiphenylphosphine (0.12 mmol) and cupric trifluoromethylsulphonate (0.3 mmol). The autoclave was heated to a temperature of 40 °C and kept at this temperature for 1 h.

The rate of dimerization of ethylene was 1300 mol per gram-atom palladium per h, with a selectivity to 1-butene of 79%.

Example 6

An experiment was carried out as described in Example 5 with the difference that 0.15 mmol of cupric trifluoromethylsulphonate was used instead of 0.3 mmol thereof. The autoclave was heated to a temperature of 50 °C and kept at this temperature for 1 h.

The rate of dimerization of ethylene was 370 mol per gram-atom palladium per h, with a selectivity to 1-butene of 94%.

**Claims**

1. Process for the preparation of a butene by dimerization of ethylene in which process ethylene is contacted in an aprotic solvent with a catalytic system, characterized in that 1-butene is prepared by using a catalytic system which is obtainable by combining the following components:-
   component (a)
   - a palladium compound,
   component (b)
   - an anion of an acid having a pKa of less than 2, measured at 18 °C in aqueous solution, with the proviso that said acid is not a hydrohalogenic acid, and
   component (c) -
   a compound containing one phosphorus and one or more nitrogen atoms which atoms bear no hydrogen atoms, and in which compound each nitrogen atom is connected to the phosphorus atom by an organic bridging group containing at least one carbon atom in the bridge.

2. A process as claimed in claim 1 in which the palladium compound is a palladium alkanoate having in the range of from 1 to 12 carbon atoms per molecule.

3. A process as claimed in claim 1 or 2 in which the acid having a pKa of less than 2 is a sulphonic acid.

4. A process as claimed in claim 3 in which the acid is trifluoromethanesulphonic acid.

5. A process as claimed in any one of the preceding claims in which component (b) is incorporated in the catalytic system in the form of an acid and/or as a non-noble transition metal salt.

6. A process as claimed in claim 5 in which component (b) is incorporated in the catalytic system as a cupric salt.

7. A process as claimed in any one of the preceding claims in which the organic bridging group in component (c) contains not more than three carbon atoms in the bridge.

8. A process as claimed in any one of the preceding claims in which component (c) may be regarded as being derived from triphenylphosphine by replacing at least one of the phenyl groups therein by a group containing one or more nitrogen atoms.

9. A process as claimed in claim 8 in which component (c) may be regarded as being derived from triphenylphosphine by replacing no more than one of the phenyl groups therein by a group containing only one nitrogen atom.

10. A process as claimed in claim 9 in which component (c) is 2-cyanoethyldiphenylphosphine, 2-pyridyldiphenylphospine or 3-(diphenylphosphino)-N,N-dimethylpropionamide.

11. A process as claimed in any one of the preceding claims in which use is made of an amount of palladium in the range of from 0.1 to 100 milligram-atom per l solvent, a molar ratio of component (b) to component (a) in the range of from 1 to 50 and a molar ratio of component (c) to component (a) in the range of from 0.1 to 10.

12. A process as claimed in any one of the preceding claims which is carried out at a temperature in the range of from 20 °C to 150 °C and a pressure in the range of from 5 to 200 bar.

**Revendications**

1. Procédé pour la préparation d'un butène par dimérisation d'éthylène, procédé dans lequel l'éthylène est mis en contact dans un solvant aprotique avec un système catalytique, caractérisé en ce qu'on prépare du 1-butène en utilisant un système catalytique qui peut s'obtenir en combinant les constituants suivants :

constituant (a) : un composé du palladium,

constituant (b) : un anion d'un acide ayant un pKa de moins de 2, mesuré à 18°C en solution aqueuse, avec la condition que cet acide n'est par un acide halogénhydrique, et

constituant (c) : un composé contenant un atome de phosphore et un ou plusieurs atomes d'azote, ces atomes ne portant pas d'atome d'hydrogène et chaque atome d'azote dans ce composé étant relié à l'atome de phosphore par un groupe organique formant pont qui contient au moins un atome de carbone dans le pont.

2. Un procédé selon la revendication 1, dans lequel le composé du palladium est un alcanoate de palladium ayant de 1 à 12 atomes de carbone par molécule.

3. Un procédé selon la revendication 1 ou 2, dans lequel l'acide ayant un pKa de moins de 2 est un acide sulfonique.

4. Un procédé selon la revendication 3, dans lequel l'acide est l'acide trifluorométhanesulfonique.

5. Un procédé selon l'une quelconque des revendications précédentes, dans lequel le constituant (b) est incorporé dans le système catalytique sous la forme d'un acide et/ou sous la forme d'un sel d'un métal de transition nonnoble.

6. Un procédé selon la revendication 5, dans lequel le constituant (b) est incorporé dans le système catalytique sous la forme d'un sel cuivrique.

7. Un procédé selon l'une quelconque des revendications précédentes, dans lequel le groupe organique formant pont dans le constituant (c) ne contient pas plus de trois atomes de carbone dans le pont.

8. Un procédé selon l'une quelconque des revendications précédentes, dans lequel le constituant (c) peut être considéré comme étant dérivé de triphénylphosphine par remplacement d'au moins un des groupes phényle de cette dernière par un groupe contenant un ou plusieurs atomes d'azote.

9. Un procédé selon la revendication 8, dans lequel le constituant (c) peut être considéré comme étant dérivé de triphénylphosphine par remplacement de pas plus d'un des groupes phényle de cette dernière par un groupe ne contenant qu'un seul atome d'azote.

10. Un procédé selon la revendication 9, dans lequel le constituant (c) est la 2-cyanoéthyldiphénylphosphine, la 2-pyridyldiphénylphosphine ou le 3-(diphénylphosphino)-N,N-diméthylpropionamide.

11. Un procédé selon l'une quelconque des revendications précédentes, dans lequel on utilise une quantité de palladium comprise entre 0,1 et 100 milliatomes-grammes par litre de solvant, un rapport molaire du constituant (b) au constituant (a) compris entre 1 et 50 et un rapport molaire du constituant (c) au constituant (a) compris entre 0,1 et 10.

12. Un procédé selon l'une quelconque des revendications précédentes, qui est mis en oeuvre à une température comprise entre 20°C et 150°C et une pression comprise entre 5 et 200 bars.

**Patentansprüche**

1. Verfahren zur Herstellung eines Butens durch Dimerisieren von Ethylen, wobei bei dem Verfahren Ethylen in einem aprotischen Lösungsmittel mit einem katalytischen System zusammengebracht wird, dadurch gekennzeichnet, daß 1-Buten hergestellt wird unter Verwendung eines katalytischen Systems, das erhältlich ist durch Kombinieren der folgenden Komponenten:

Komponente (a) -
eine Palladiumverbindung,

Komponente (b) -
ein Anion einer Säure mit einem pKa-Wert von kleiner 2, gemessen bei 18°C in wäßriger Lösung, mit der Maßgabe, daß die Säure keine Halogenwasserstoffsäure ist, und

Komponente (c) -
eine Verbindung, enthaltend ein Phosphor- und ein oder mehrere Stickstoffatome, wobei die Atome keine Wasserstoffatome tragen und wobei in der Verbindung jedes Stickstoffatom mit dem Phosphoratom über eine organische Brückengruppe, enthaltend mindestens ein Kohlenstoffatom in der Brücke, verbunden ist.

2. Verfahren nach Anspruch 1, wobei die Palladiumverbindung ein Palladiumalkanoat mit 1 bis 12 Kohlenstoffatomen pro Molekül ist.

3. Verfahren nach Anspruch 1 oder 2, wobei die Säure mit einem pKa-Wert kleiner 2 eine Sulfonsäure ist.

4. Verfahren nach Anspruch 3, wobei die Säure Trifluormethansulfonsäure ist.

5. Verfahren nach einem der vorangehenden Ansprüche, wobei die Komponente (b) in das katalytische System in Form einer Säure und/oder eines nicht-Edelmetall-Übergangsmetallsalzes eingebaut ist.

6. Verfahren nach Anspruch 5, wobei die Komponente (b) in das katalytische System als Kupfersalz eingebaut ist.

7. Verfahren nach einem der vorangehenden Ansprüche, wobei die organische Brückengruppe in der Komponente (c) nicht mehr als 3 Kohlenwasserstoffatome in der Brücke enthält.

8. Verfahren nach einem der vorangehenden Ansprüche, wobei Komponente (c) angesehen werden kann als abgeleitet von Triphenylphosphin durch Ersatz mindestens einer der Phenylgruppen durch eine Gruppe, enthaltend ein oder mehrere Stickstoffatome.

9. Verfahren nach Anspruch 8, wobei die Komponente (c) angesehen werden kann als abgeleitet von Triphenylphosphin durch Ersatz von nicht mehr als einer der Phenylgruppen durch eine Gruppe, enthaltend nur ein Stickstoffatom.

10. Verfahren nach Anspruch 9, wobei die Komponente (c) 2-Cyanoethyldiphenylphosphin, 2-Pyridyldiphenylphosphin oder 3-(Diphenylphosphino)-N,N-dimethylpropionamid ist.

11. Verfahren nach einem der vorangehenden Ansprüche, wobei eine Menge an Palladium im Bereich von 0,1 bis 100 mgAtom pro Liter Lösungsmittel verwendet wird, ein Molverhältnis von Komponente (b) zu Komponente (a) im Bereich von 1 bis 50 und ein Molverhältnis von Komponente (c) zu Komponente (a) im Bereich von 0,1 bis 10 angewandt wird.

12. Verfahren nach einem der vorangehenden Ansprüche, das bei einer Temperatur im Bereich von 20°C bis 150°C und bei einem Druck im Bereich von 5 bis 200 bar durchgeführt wird.